# EUROPEAN PATENT SPECIFICATION

(11) **EP 1 854 500 B1**
(45) Date of publication and mention of the grant of the patent: **30.03.2016**
(21) Application number: 07009065.9
(22) Date of filing: 04.05.2007
(51) Int. Cl.: A61M 25/01, A61M 25/00

(54) **Treatment tool inserting/withdrawing auxiliary device**
Hilfsvorrichtung zur Einführung/Entnahme eines Behandlungswerkzeugs
Dispositif auxiliaire d'insertion/de retrait d'un outil de traitement

(30) Priority: 09.05.2006 US 430739
(43) Date of publication of application: 14.11.2007
(73) Proprietor: Olympus Corporation, Shibuya-ku Tokyo 151-0072 (JP); Terumo Kabushiki Kaisha, Shibuya-ku Tokyo (JP)
(72) Inventor: Miyamoto, Satoshi, Shibuya-ku Tokyo (JP); Motai, Kousuke, Shibuya-ku Tokyo (JP); Nishiie, Takehiro, Shibuya-ku Tokyo (JP); Muramatsu, Junichi, Shibuya-ku Tokyo (JP); Kuroda, Yasuyuki, Kanagawa-ken (JP); Kousai, Tadashi, Shizuoka-ken (JP); Kobayashi, Junichi, Shizuoka-ken (JP); Kinoshita, Yasushi, Fujinomiya-shi, Shizuoka-ken (JP)
(74) Representative: von Hellfeld, Axel

(56) References cited:
- WO-A-2006/042739
- US-A- 4 214 593
- US-A- 4 577 621
- US-A- 5 372 587
- US-A- 5 599 300
- US-A- 5 961 511
- US-A1- 2005 090 847

## Description

### BACKGROUND OF THE INVENTION

### Field of the Invention

The present invention relates to a treatment tool inserting/withdrawing auxiliary device.

### Description of Related Art

For performing cannulation (selective insertion into a pancreatic duct/bile duct) in diagnosis and treatment of a pancreatic/bile duct system, while a treatment tool such as a contrast medium injecting catheter is inserted into a channel of a flexible endoscope, back-and-forth moving operation, angling operation, and twisting operation of the endoscope insertion portion, raising-up operation of a forcep stage arranged on the distal end of the insertion portion, and back-and-forth moving operation of the contrast medium injecting catheter with respect to the channel are performed in combination. In this case, it is necessary to match the axial directions of the distal end of the contrast medium injecting catheter and the bile duct (or the pancreatic duct) by delicate operations of the endoscope and the contrast medium injecting catheter.

Therefore, a contrast medium injecting catheter for facilitating these operations is disclosed in Japanese Unexamined Patent Application, First Publication No. 2002-272675 and Published Japanese Translation No. 2004-532668 of PCT International Publication. Moreover, in a medical procedure other than for the bile duct system, there is an attempt to facilitate the manipulation by means of a catheter as disclosed in U.S. Patent No. 6,659,981.

According to these, since the distal end of the catheter is curved to some extent, the positioning operation by the endoscope insertion portion can be assisted.

On the other hand, after the cannulation, in order to exchange the contrast medium injecting catheter and a treatment tool required for the subsequent treatment, a guide wire having at least twice the length of the contrast medium injecting catheter is inserted into the contrast medium injecting catheter, and the contrast medium injecting catheter is withdrawn from the endoscope through the guide wire. Then, the treatment tool to be used is inserted into the endoscope along the guide wire, and moved to the target site.

However, because the operation of bringing the endoscope to a position where cannulation can be readily performed cannot be perceptively performed, the distal end of the catheter must be positioned by a skilled manoeuvering of the endoscope even though the distal end of the catheter is curved to some extent. As a result, a high skill for positioning is required for the operator.

Moreover, when plural treatment tools are used, the treatment tools must be inserted and withdrawn using a guide wire; therefore, the exchanging operation between the treatment tools is complicated, and makes the operation time longer.

US 2005/0090847 A1 relates to an apparatus for, and a method of, accurate positioning of endoscopic instruments. Accurate positioning of the instruments is accomplished through the inclusion of a steering ability within the device. After the endoscopic instrument is properly positioned a rapid exchange technology, soft locks, and mechanical locks may be used to maintain the position of the endoscopic instrument. Rapid exchange technology is used to minimize displacement forces present on the guidewire or catheters. Soft locks and mechanical locks resist movements caused by displacement forces.

### SUMMARY OF THE INVENTION

An object of the present invention is to provide a device capable of readily performing cannulation while the distal end of an endoscope insertion portion is positioned with respect to the duodenal papilla, and capable of exchanging treatment tools without using a guide wire.

A treatment tool inserting/withdrawing auxiliary device according to the present invention is defined by the independent claim.

Preferred embodiments of treatment tool inserting / with drawing auxiliary device are defined by the dependent claims.

According to the present invention, because the distal end of the catheter projecting from the channel of the endoscope can be brought close to the proximal portion of the curvable catheter so that the direction of the distal end of the catheter and the direction of the proximal portion define a predetermined angle, the direction of the distal end of the catheter can be matched with the direction of insertion by merely curving the curvable portion of the curvable catheter. Accordingly, without requiring a high skill for positioning, cannulation can be readily performed in a condition where the distal end of the endoscope insertion portion is fixed.

### BRIEF DESCRIPTION OF THE DRAWINGS

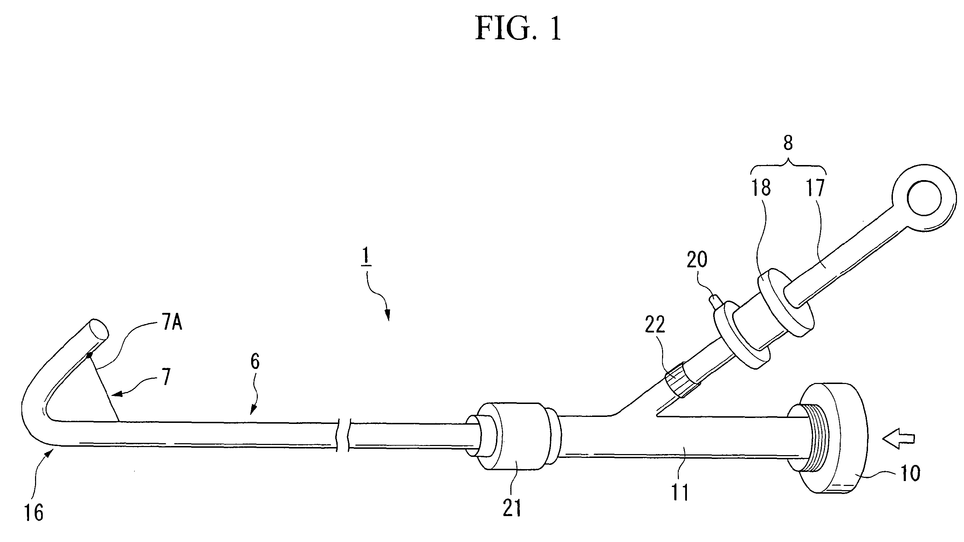

FIG. 1 is an overall schematic diagram showing a treatment tool inserting/withdrawing auxiliary device according to a first exemplary embodiment.

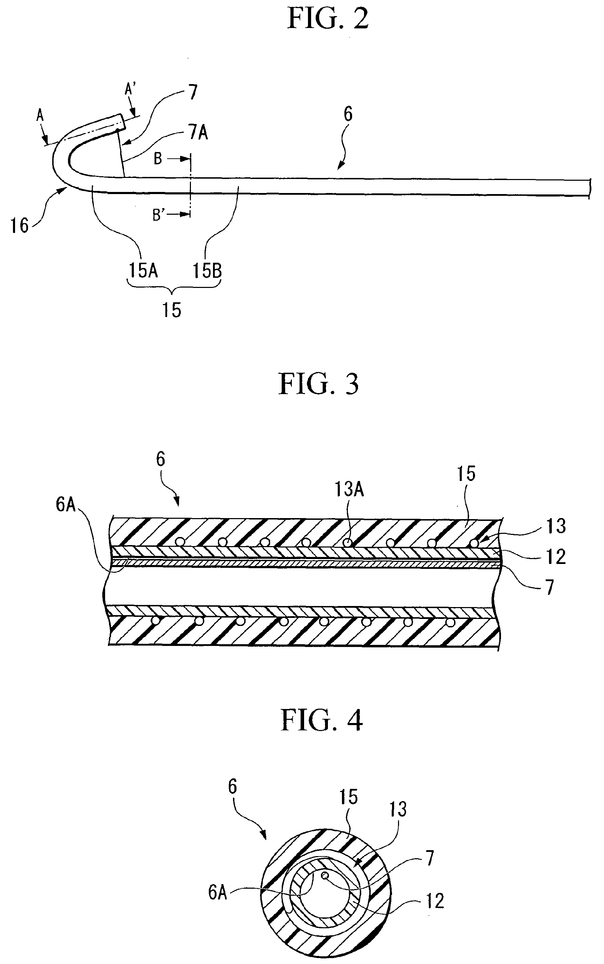

FIG. 2 is a main part side view showing a curvable catheter of the treatment tool inserting/withdrawing auxiliary device according to the first exemplary embodiment.
FIG. 3 is a cross-sectional view taken along the line A-A' in FIG. 2.
FIG. 4 is a cross-sectional view taken along the line B-B' in FIG. 2.

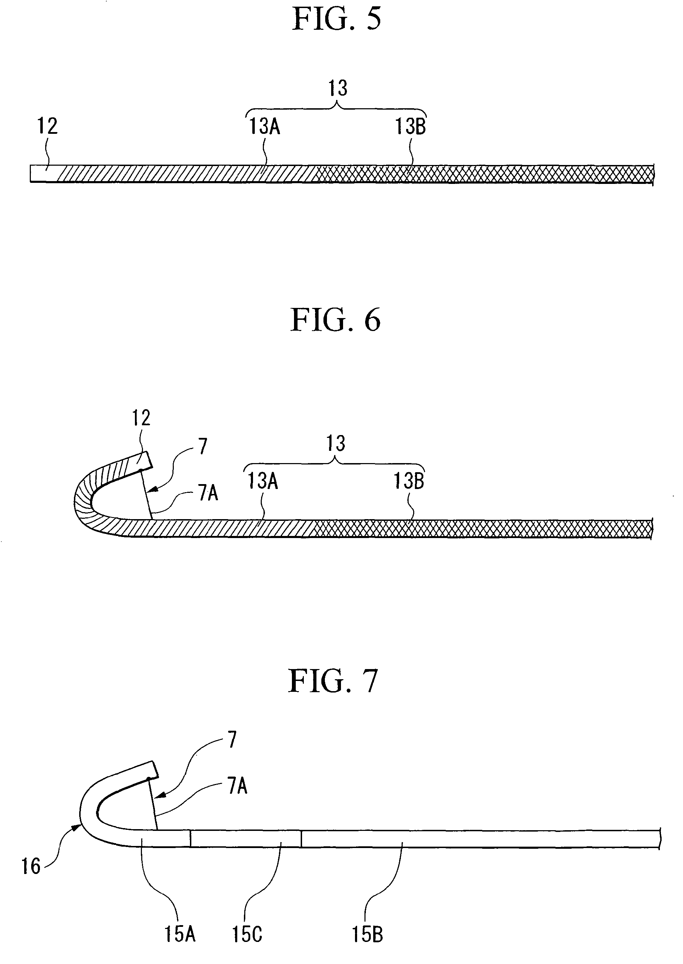

FIG. 5 is an internal configuration diagram of the main part showing the curvable catheter of the treatment tool inserting/withdrawing auxiliary device according to the first exemplary embodiment.
FIG. 6 is an internal configuration diagram of the main part showing the curvable catheter of the treatment tool inserting/withdrawing auxiliary device according to the first exemplary embodiment.
FIG. 7 is an internal configuration diagram of the main part showing a modified example of the curvable catheter of the treatment tool inserting/withdrawing auxiliary device according to the first exemplary embodiment.

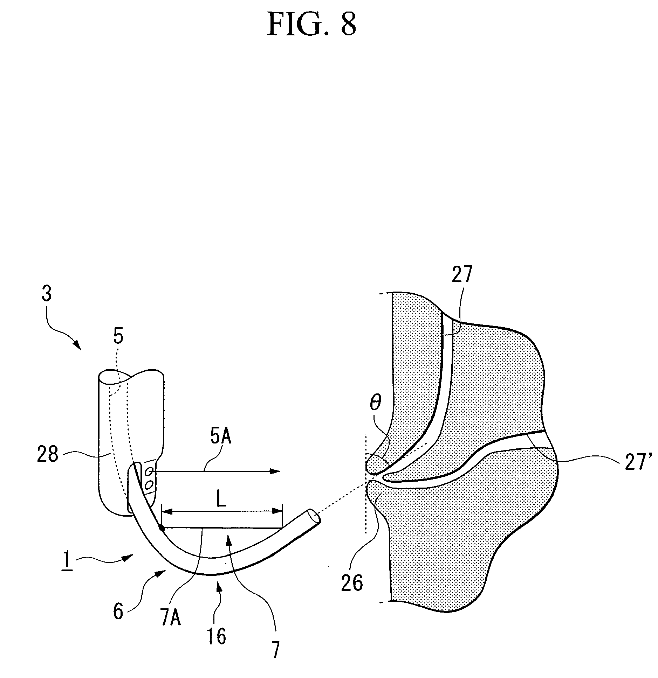

FIG. 8 is an explanatory diagram showing the curved condition of the curvable catheter of the treatment tool inserting/withdrawing auxiliary device according to the first exemplary embodiment.
FIG. 9 is a cross-sectional view showing a branched portion of the treatment tool inserting/withdrawing auxiliary device according to the first exemplary embodiment.
FIG. 10 is a cross-sectional view showing a modified example of the branched portion of the treatment tool inserting/withdrawing auxiliary device according to the first exemplary embodiment.

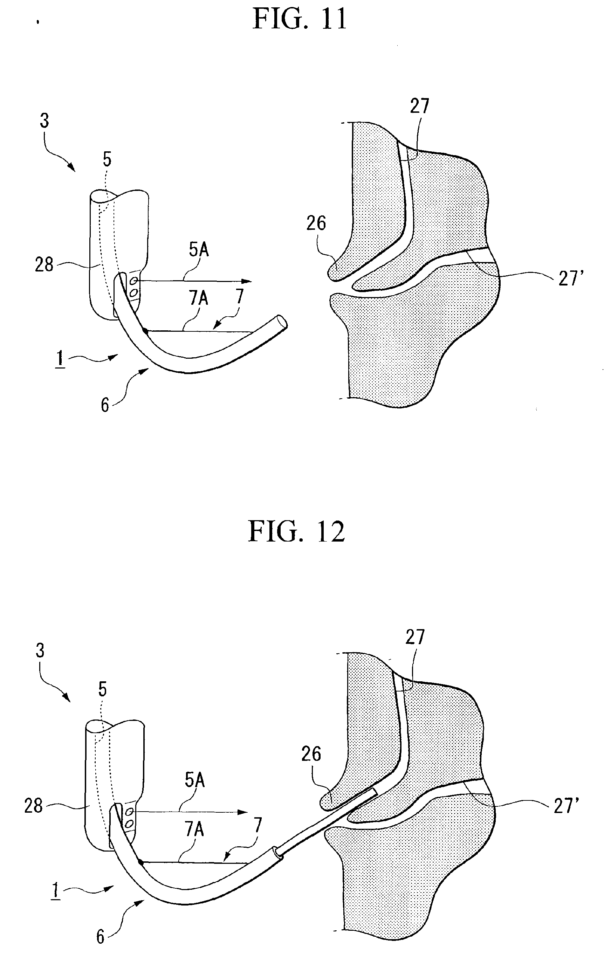

FIG. 11 is an explanatory diagram showing a medical procedure through an endoscope by the treatment tool inserting/withdrawing auxiliary device according to the first exemplary embodiment.
FIG. 12 is an explanatory diagram showing the medical procedure through the endoscope by the treatment tool inserting/withdrawing auxiliary device according to the first exemplary embodiment.

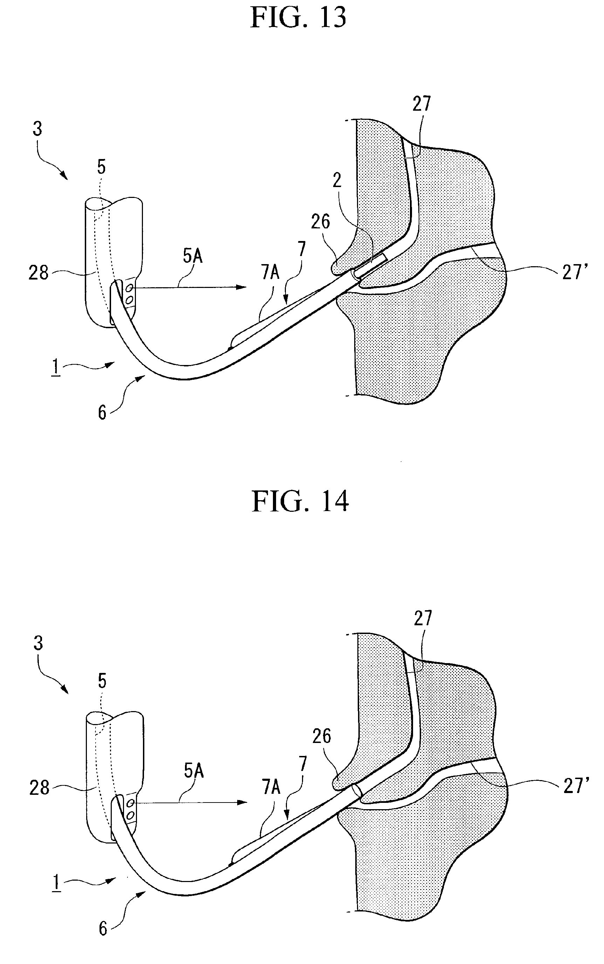

FIG. 13 is an explanatory diagram showing the medical procedure through the endoscope by the treatment tool inserting/withdrawing auxiliary device according to the first exemplary embodiment.
FIG. 14 is an explanatory diagram showing the medical procedure through the endoscope by the treatment tool inserting/withdrawing auxiliary device according to the first exemplary embodiment.
FIG. 15 is an explanatory diagram showing the medical procedure through the endoscope by the treatment tool inserting/withdrawing auxiliary device according to the first exemplary embodiment.
FIG. 16 is an internal configuration diagram of the main part showing the curvable catheter of the treatment tool inserting/withdrawing auxiliary device according to a second exemplary embodiment.
FIG. 17 is an explanatory diagram showing a curved condition of the curvable catheter of the treatment tool inserting/withdrawing auxiliary device according to a third exemplary embodiment.
FIG. 18 is a main part plan view showing the curvable catheter of the treatment tool inserting/withdrawing auxiliary device according to the third exemplary embodiment.
FIG. 19 is an overall schematic diagram showing the treatment tool inserting/withdrawing auxiliary device according to a fourth embodiment.
FIG. 20 is a main part plan view showing the curvable catheter of the treatment tool inserting/withdrawing auxiliary device according to a fifth exemplary embodiment.
FIG. 21 is a main part plan view showing a modified example of the curvable catheter of the treatment tool inserting/withdrawing auxiliary device according to the fifth exemplary embodiment.
FIG. 22 is an explanatory diagram showing a curved condition of the curvable catheter of the treatment tool inserting/withdrawing auxiliary device according to the fifth exemplary embodiment.
FIG. 23 is an explanatory diagram showing a medical procedure through an endoscope by the treatment tool inserting/withdrawing auxiliary device according to the fifth exemplary embodiment.
FIG. 24 is an explanatory diagram showing the curved condition of the treatment tool inserting/withdrawing auxiliary device according to the fifth exemplary embodiment.
FIG. 25 is an explanatory diagram showing the medical procedure through the endoscope by the treatment tool inserting/withdrawing auxiliary device according to the fifth exemplary embodiment.
FIG. 26 is an explanatory diagram showing a curved condition of the curvable catheter of the treatment tool inserting/withdrawing auxiliary device according to a sixth exemplary embodiment.
FIG. 27 is a main part plan view showing the curvable catheter of the treatment tool inserting/withdrawing auxiliary device according to a seventh exemplary embodiment.
FIG. 28 is a main part cross-sectional view showing the curvable catheter of the treatment tool inserting/withdrawing auxiliary device according to the seventh exemplary embodiment.
FIG. 29 is an explanatory diagram showing a medical procedure through an endoscope by a modified example of the treatment tool inserting/withdrawing auxiliary device.
FIG. 30 is an explanatory diagram showing the medical procedure through the endoscope by the modified example of the treatment tool inserting/withdrawing auxiliary device.
FIG. 31 is a main part side view showing the curvable catheter of the modified example of the treatment tool inserting/withdrawing auxiliary device.
FIG. 32 is a main part cross-sectional view showing the curvable catheter of the modified example of the treatment tool inserting/withdrawing auxiliary device.

### DESCRIPTION OF THE PREFERRED EMBODIMENTS

Hereunder is a detailed description of preferred exemplary embodiments useful for understanding the present invention and a preferred embodiment according to the present invention. In the following description, the same reference symbols are used for the same components, and duplicate descriptions are omitted.

### (First exemplary embodiment)

A treatment tool inserting/withdrawing auxiliary device 1 according to the present exemplary embodiment includes: a curvable catheter (catheter) 6 having a through hole 6A through which a contrast medium injecting catheter (treatment tool for an endoscope, first treatment tool) 2 can be inserted, and which can be inserted into a channel 5 of a flexible endoscope 3; an operation wire 7 which is inserted into the curvable catheter 6, and is partially exposed to the outside of the curvable catheter 6 to form an exposed portion 7A supported on a distal end of the curvable catheter 6; an operation portion 8 which moves the operation wire 7 back and forth with respect to the curvable catheter 6; a treatment tool insertion/withdrawal port 10 which is provided in communication with the curvable distance of the slider 18 with respect to the operation portion mainbody 17 has a sufficient length for the angle θ to be changed from 10 degrees to 90 degrees.

The operation portion 8 is provided with a ratchet mechanism (not shown), enabling to move the slider 18 only to the proximal side of the operation portion mainbody 17. By pressing a release button 20 provided on the slider 18, the slider 18 can be also moved to the distal side of the operation portion mainbody 17.

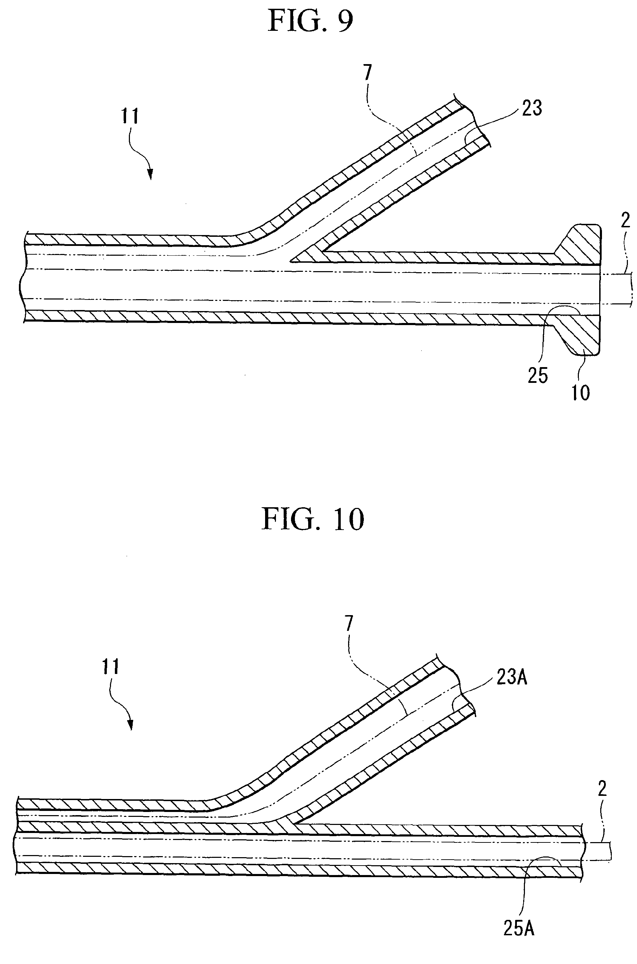

The branched portion 11 includes a first connector 21 which is detachably connected to the proximal end of the curvable catheter 6, and a second connector 22 which is detachably connected to the operation portion mainbody 17. Moreover, as shown in FIG. 9, the branched portion 11 is provided with: a first through hole 23 which is communicated with the through hole 6A, and is inserted with the operation wire 7 arranged in the curvable catheter 6; and a second through hole 25 which is communicated with the through hole 6A, and inserted with an endoscope treatment tool such as the contrast medium injecting catheter 2, branched in the middle. As shown in FIG. 10, a first through hole 23A and a second through hole 25A may be respectively and separately extended to the distal end of the curvable catheter.

Next is a description of the effect of the treatment tool inserting/withdrawing auxiliary device 1 according to the present exemplary embodiment, together with a medical procedure through an endoscope using this. As the following medical procedure, the description is regarding a manipulation such as inserting the contrast medium injecting catheter 2 into a duodenal papilla 26 using the endoscope 3, injecting a contrast medium into a bile duct 27 to diagnose under X-ray fluoroscopy, and removing all bile duct calculi. For a manipulation regarding the pancreatic duct 27', in principle, the bile duct 27 is replaced with a pancreatic duct 27' in the following description. into/from the channel 5 can be readily performed, and the rotation torque when rotated with respect to the channel 5 can be suitably transferred to the distal side.

Moreover, the first resin layer 12 serving as the innermost layer of the curvable catheter 6 contains a fluororesin. Therefore the frictional force can be reduced, and the contrast medium injecting catheter 2 and another endoscope treatment tool inserted into the through hole 6A can be smoothly inserted/withdrawn. The first resin layer may contain a hydrophilic resin.

Moreover, in the example of inserting the contrast medium injecting catheter 2, the effect of the treatment tool inserting/withdrawing auxiliary device 1 was described. However, another treatment tool for an endoscope 29 such as a balloon may be inserted instead of the contrast medium injecting catheter 2 at the beginning.

### (Second exemplary embodiment)

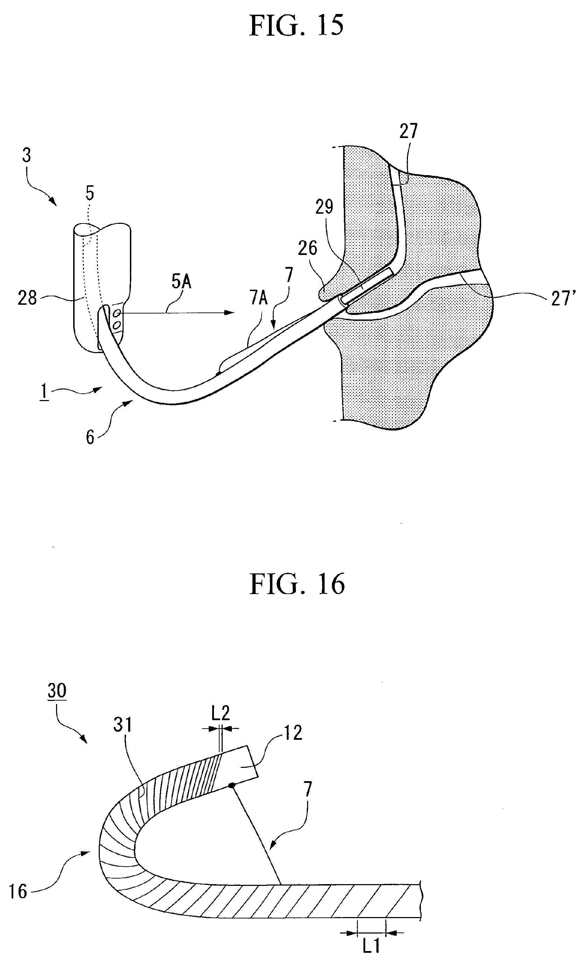

A second exemplary embodiment useful for understanding the present invention is described with reference to the drawings. The difference between the second exemplary embodiment and the first exemplary embodiment is the point that, as shown in FIG. 16, a treatment tool inserting/withdrawing auxiliary device 30 according to the present exemplary embodiment has a pitch L1 on the proximal side of the coil layer 31 greater than a pitch L2 on the distal side thereof.

The pitch L2 in the vicinity of the curvable portion 16 has the same interval as that of the coil layer 13A according to the first exemplary embodiment. The pitch L1 in the connection part with the mesh pipe 13B is 0.5mm to 0.6mm, and the pitch in the middle is changed so that the pitch gradually becomes greater from the distal side to the proximal side.

According to this treatment tool inserting/withdrawing auxiliary device 30 and medical procedure through an endoscope, a similar effect to that of the first exemplary embodiment can be demonstrated. Moreover, by using this device, a similar medical procedure can be performed. In particular, since the pitch of the coil layer 31 is small on the distal side in the vicinity of the curvable portion 16, the curvable portion 16 can be curved without buckling. Moreover, since the pitch is gradually changed, in the connection part between the coil layer 31 and the mesh pipe 13B, discontinuous change with respect to the curve rigidity can be made less than that of the first embodiment, and the buckling resistance can be improved.

### (Third exemplary embodiment)

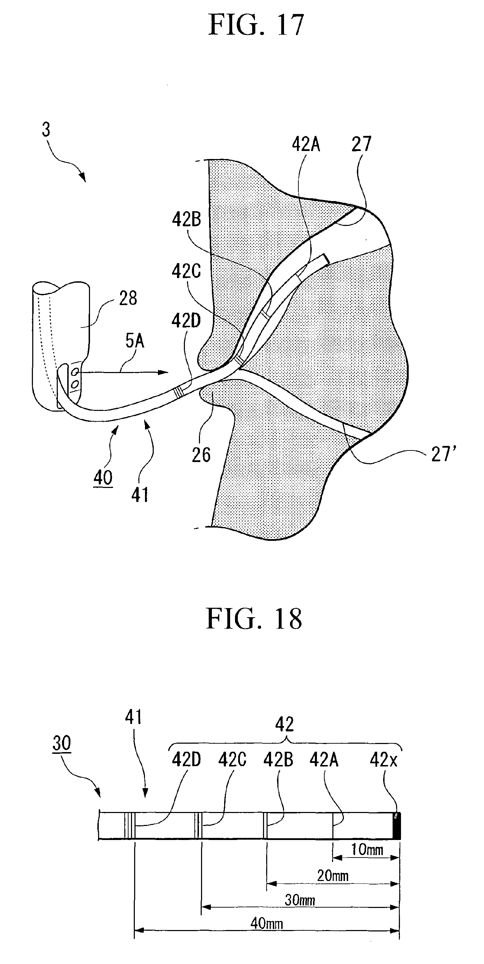

A third exemplary embodiment useful for understanding the present invention is described with reference to the drawings. The difference between the third exemplary embodiment and the first exemplary embodiment is the point that, as shown in FIG. 17 and FIG. 18, the distal end of a curvable catheter 41 of a treatment tool inserting/withdrawing auxiliary device 40 according to the present exemplary embodiment is provided with visual check markers 42 for identifying the length from the distal end.

The visual check markers 42 include for example: a reference visual check marker 42X provided at the most distal end of the curvable catheter 41; a first position 42A provided in a position 10mm from the distal end of the curvable catheter 41; a second position 42B provided in a position 20mm therefrom; a third position 42C provided in a position 30mm therefrom; and a fourth position 42D provided in a position 40mm therefrom. The respective positions may be provided by an X-ray impermeable material so as to be observable under X-ray contrast radiography. Moreover, the number of the provided markers and the distance from the distal end of the curvable catheter 41 are not limited to the above, and may be provided according to the manipulation.

According to this treatment tool inserting/withdrawing auxiliary device 40 and medical procedure through an endoscope, since the visual check markers 42 are provided, it can be ascertained in an X-ray image under X-ray contrast radiography how far the distal side of the curvable catheter 41 is inserted into the bile duct and the like. Moreover, even if not under X-ray contrast radiography, the visual check markers 42 can be confirmed by an endoscopic image.

### (Fourth embodiment)

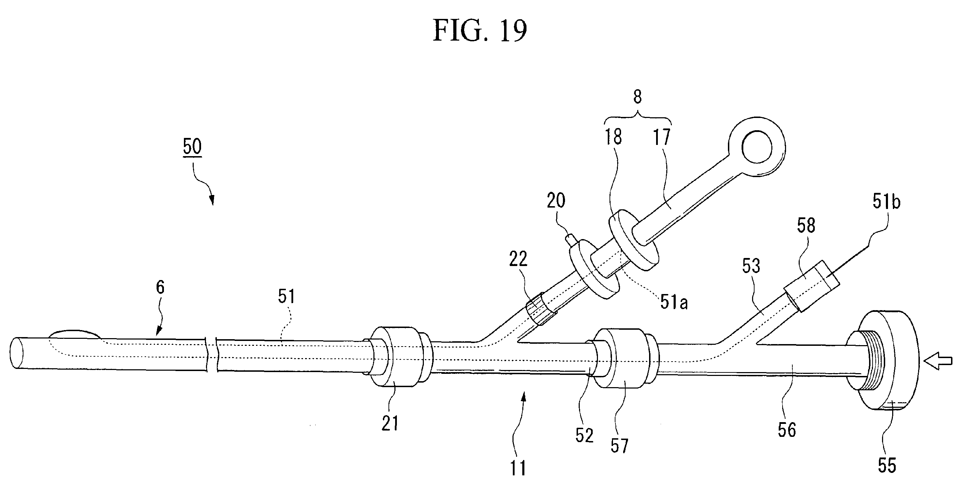

A fourth embodiment of the present invention is described with reference to the drawings. The difference between the fourth embodiment and the first exemplary embodiment is the point that, as shown in FIG. 19, an operation wire 51 of a treatment tool inserting/withdrawing auxiliary device 50 according to the present embodiment has one end 51 a connected to the operation portion mainbody 17 of the operation portion 8, and the other end 51b side folded at the distal end of the curvable catheter 6 and arranged toward the proximal side.

To a treatment tool insertion/withdrawal port 52 of the branched portion 11 is detachably connected an extension portion 56 that is provided with a new treatment tool insertion port 55 and a wire insertion/withdrawal port 53 through which the other end 51 b side of the operation wire 51 is inserted, via a connector 57. While the extension portion 56 is connected, the second through hole 25 of the branched portion 11, the wire insertion/withdrawal port 53, and the new treatment tool insertion/withdrawal port 55 are communicated.

The operation wire 51 projecting from the wire insertion/withdrawal port 53 is arranged with a clasp 58 for preventing the operation wire 51 from being pulled into the wire insertion/withdrawal port 53. This clasp 58 is formed larger than the inner diameter of the wire insertion/withdrawal port 53, and detachably attached to the operation wire 51.

Next is a description of the effect of the treatment tool inserting/withdrawing auxiliary device 50 according to the present embodiment, together with a medical procedure through an endoscope using this.

First, similarly to the first exemplary embodiment, the curvable catheter 6 of the treatment tool inserting/withdrawing auxiliary device 50 is made to project from the distal opening of the channel of an endoscope (not shown).

Next, an operator (not shown) performs a similar operation to that of the first exemplary embodiment while observing an observation image. In a condition where the curvable portion 16 is curved at a predetermined angle to match the distal direction of the curvable catheter 6 with the direction of the bile duct (not shown), the contrast medium injecting catheter (not shown) is inserted into the bile duct. Then, the contrast medium is poured into the contrast medium injecting catheter, and the inside of the bile duct is visually observed by means of X-ray contrast radiography.

When the curvable catheter 6 is inserted into the bile duct, the clasp 58 is taken off from the operation wire 51, and further the connector 22 is separated to take out the operation portion 8 from the curvable catheter 6. At this time, the other end 51 b side of the operation wire 51 is moved to the distal direction in the curvable catheter 6, is folded at the distal end, and goes again toward the proximal side, to be pulled out from the curvable catheter 6 together with the operation portion 8.

The curvable catheter 6 is inserted into the bile duct, and after withdrawing the operation wire 51 and the operation portion 8, the contrast medium injecting catheter 2 is withdrawn from the treatment tool insertion/withdrawal port 55 of the curvable catheter 6. The treatment tool insertion/withdrawal port 55 of the curvable catheter 6 is inserted with another treatment tool for an endoscope such as a balloon (not shown), instead, to perform a predetermined treatment on the target site.

According to this treatment tool inserting/withdrawing auxiliary device 50 and medical procedure through an endoscope, the operation wire 51 can be withdrawn from the curvable catheter 6 after the contrast medium injecting catheter is inserted into the bile duct. Therefore, when the endoscope treatment tool is inserted into the curvable catheter 6, the treatment tool can be more readily exchanged without being interfered with by the operation wire 51.

### (Fifth exemplary embodiment)

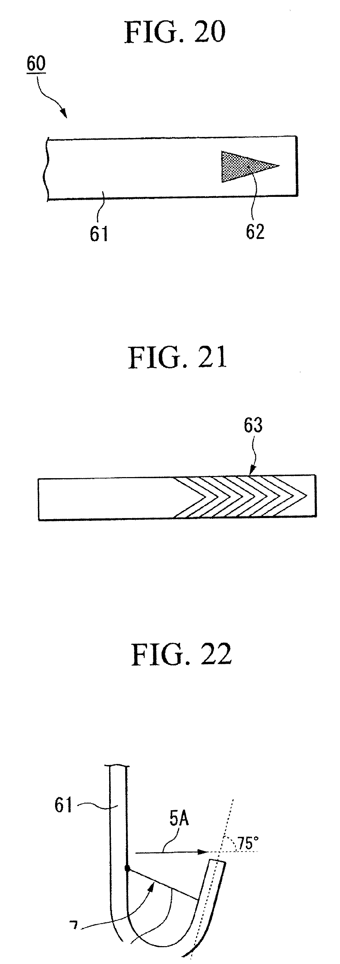

A fifth exemplary embodiment useful for understanding the present invention is described with reference to the drawings. The difference between the fifth exemplary embodiment and the first exemplary embodiment is the point that, as shown in FIG. 20, the distal outer face of a curvable catheter 61 of a treatment tool inserting/withdrawing auxiliary device 60 according to the present exemplary embodiment is provided with an index 62 formed to be gradually wider toward the proximal side.

The index 62 is formed in an approximate isosceles triangle, and is arranged in a predetermined position on the distal side from the exposed portion 7A of the operation wire 7, so that the apex of the isosceles faces to the distal side of the curvable catheter 61. The shape of the index 62 is not limited to an approximate isosceles triangle. As shown in FIG. 21, there may be an index 63 where a plurality of only the isosceles portions of isosceles triangles are arranged in a row in the longitudinal direction of the curvable catheter 61.

Next is a description of the effect of the treatment tool inserting/withdrawing auxiliary device 60 according to the present exemplary embodiment, together with a medical procedure through an endoscope using this.

First, similarly to the first exemplary embodiment, the distal end of the insertion portion of an endoscope (not shown) is positioned in the vicinity of the duodenal papilla 26, to capture the duodenal papilla 26 in the endoscope image. Next, the curvable catheter 61 of the treatment tool inserting/withdrawing auxiliary device 60 is inserted into the channel, so as to project from the distal opening of the channel.

An operator (not shown) performs a similar operation to that of the first exemplary embodiment while observing an observation image V by means of the endoscope, to curve the curvable portion 16 at a predetermined angle, so that the distal end of the curvable catheter 61 faces toward the proximal direction of the curvable catheter 61.

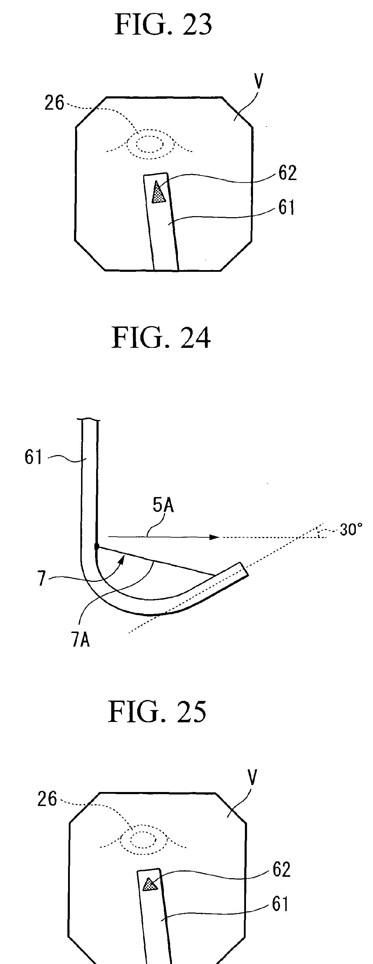

At this time, if the distal end of the curvable catheter 61 is arranged in a direction approximately orthogonal to the line of sight 5A (75 degree direction in the drawing) as shown in FIG. 22, the index 62 appears in a nearly original form of the approximate isosceles triangle in the observation image as shown in FIG. 23. On the other hand, if the distal end of the curvable catheter 61 is arranged close to the direction of the line of sight 5A (30 degree direction in the drawing) as shown in FIG. 24, the index 62 appears in a squashed form in the height direction in the observation image as shown in FIG. 25.

Here, in order to insert a contrast medium injecting catheter (not shown) into the bile duct 27, it is required that the distal end of the curvable catheter 61 faces the direction of 11 o'clock with respect to the duodenal papilla 26. In order to insert it into the pancreatic duct (not shown), it is required that the distal end of the curvable catheter 61 faces the direction of 4 o'clock with respect to the duodenal papilla 26. Therefore, the shape of the index 62 is observed to judge from the shape in the image whether or not the distal direction of the curvable catheter 61 is matched with the direction of the bile duct 27 or the pancreatic duct 27'. Then the contrast medium injecting catheter that has been inserted into the curvable catheter 61 is pushed out, and the distal end of the contrast medium injecting catheter is inserted from the duodenal papilla 26 into the bile duct 27.

According to this treatment tool inserting/withdrawing auxiliary device 60 and medical procedure through an endoscope, the shape of the index 62 in the observation image appears differently according to the curved angle of the distal end of the curvable catheter 61 with respect to the insertion portion 28. Therefore, the operator can readily judge whether or not the distal end of the curvable catheter 61 faces toward a predetermined direction by visually checking the shape of the index 62 in the observation image.

### (Sixth exemplary embodiment)

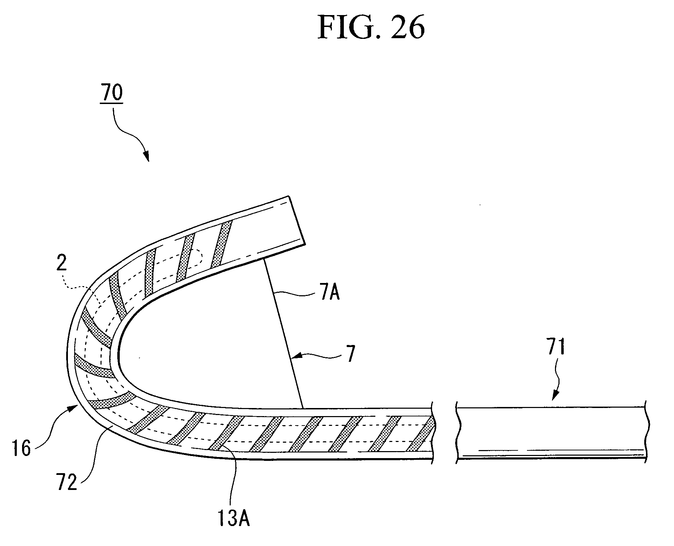

A sixth exemplary embodiment useful for understanding the present invention is described with reference to the drawings. The difference between the sixth exemplary embodiment and the first exemplary embodiment is the point that, as shown in FIG. 26, a first resin layer (not shown) and a second resin layer 72 in the region including the curvable portion 16 of a curvable catheter 71 of a treatment tool inserting/withdrawing auxiliary device 70 according to the present exemplary embodiment are made from a transparent resin.

The first resin layer and the second resin layer 72 are respectively made from resins such as nylon, urethane, PTFE, and PFA. As a result, the position of the distal end of a treatment tool such as the contrast medium injecting catheter 2 passing through the inside of the curvable catheter 71 can be visually checked in an observation image of the endoscope.

According to this treatment tool inserting/withdrawing auxiliary device 70 and medical procedure through an endoscope, in the region including the transparent resin, a treatment tool passing through the inside can be visually checked in an observation image of the endoscope, and the position of the treatment tool with respect to the curvable catheter 71 can be readily determined.

### (Seventh exemplary embodiment)

A seventh exemplary embodiment useful for understanding the present invention is described with reference to the drawings. The difference between the seventh exemplary embodiment and the first exemplary embodiment is the point that the surface of the proximal side of a curvable catheter 81 of a treatment tool inserting/withdrawing auxiliary device 80 according to the present exemplary embodiment is provided with convex portions 82.

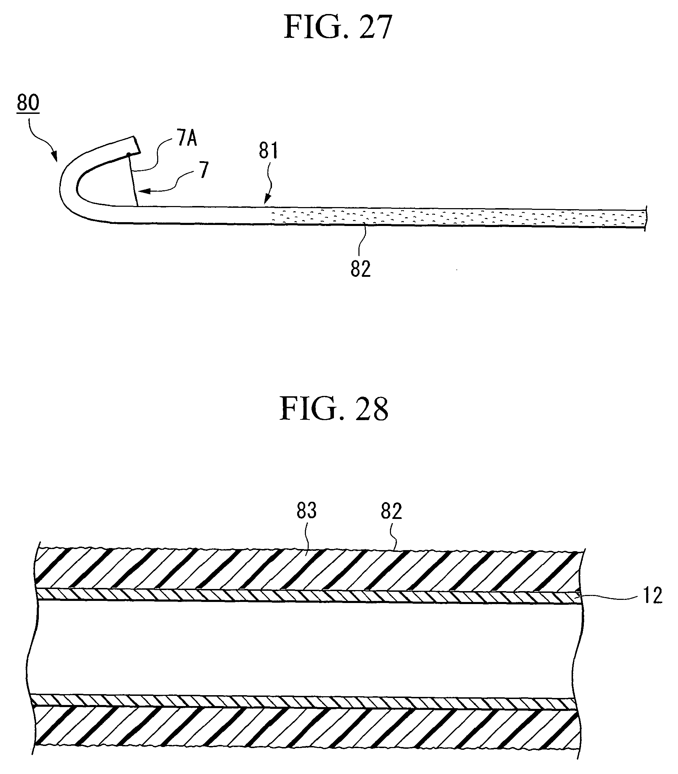

The convex portions 82 are minute and are formed on the second resin layer 83 on the proximal side from the curvable portion 16 as shown in FIG. 27 and FIG. 28. The convex portions 82 may be provided not only on the proximal side of the curvable catheter, but also to the distal end. Moreover, concavities may be provided instead of the convex portions 82.

According to this treatment tool inserting/withdrawing auxiliary device 80 and medical procedure through an endoscope, because the surface of the curvable catheter 81 is provided with the convex portions 82, when the curvable catheter 81 is inserted into a channel (not shown), the frictional force with the wall surface of the channel can be reduced and the curvable catheter 81 can be readily inserted/withdrawn. Moreover, instead of the convex portions 82, or in addition to the convex portions 82, the surface of at least the proximal side of the curvable catheter 81 may be a hydrophilic lubricant surface.

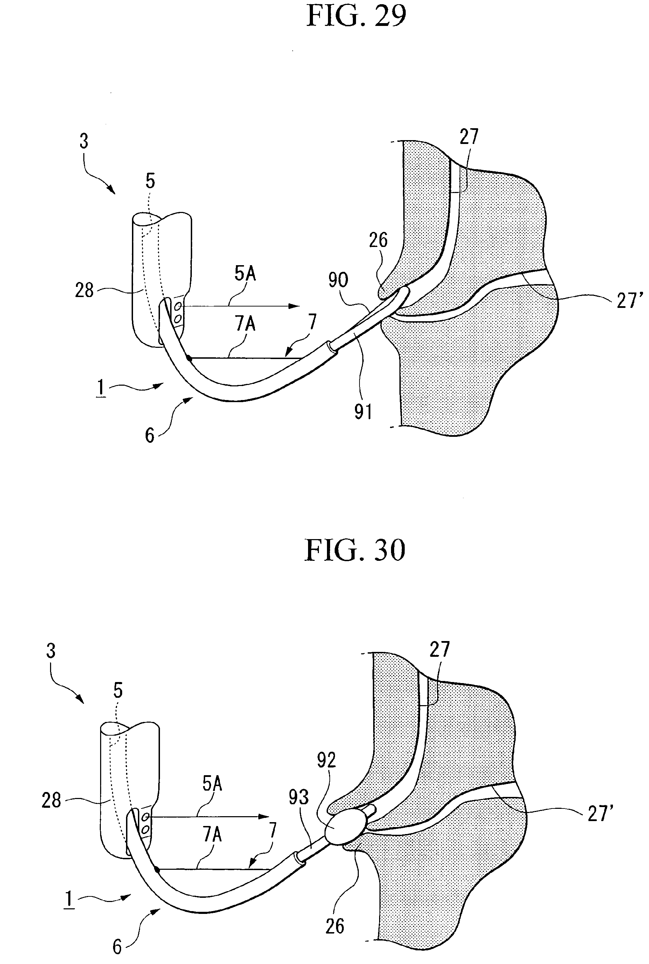

The technical scope of the present invention is not limited to the above exemplary embodiments and the above embodiment, and various modifications can be made without departing from the scope of the present invention. For example, there may be used a contrast medium injecting catheter 91 provided with an incision knife 90 connected to a high frequency power source (not shown) on the distal end as shown in FIG. 29, or a contrast medium injecting catheter 93 provided with a balloon 92 as shown in FIG. 30.

In this case, in the abovementioned medical procedure, the contrast medium is poured into the contrast medium injecting catheter 91 or 93, and the inside of the bile duct 27 is visually observed by means of X-ray contrast radiography. Then, without withdrawing the contrast medium injecting catheter 91 or 93 from the treatment tool insertion/withdrawal port (not shown) of the curvable catheter 6, the sphincter of the duodenal papilla 26 can be incised as it is by the incision knife 90, or the sphincter of the duodenal papilla 26 can be extended by the balloon 92.

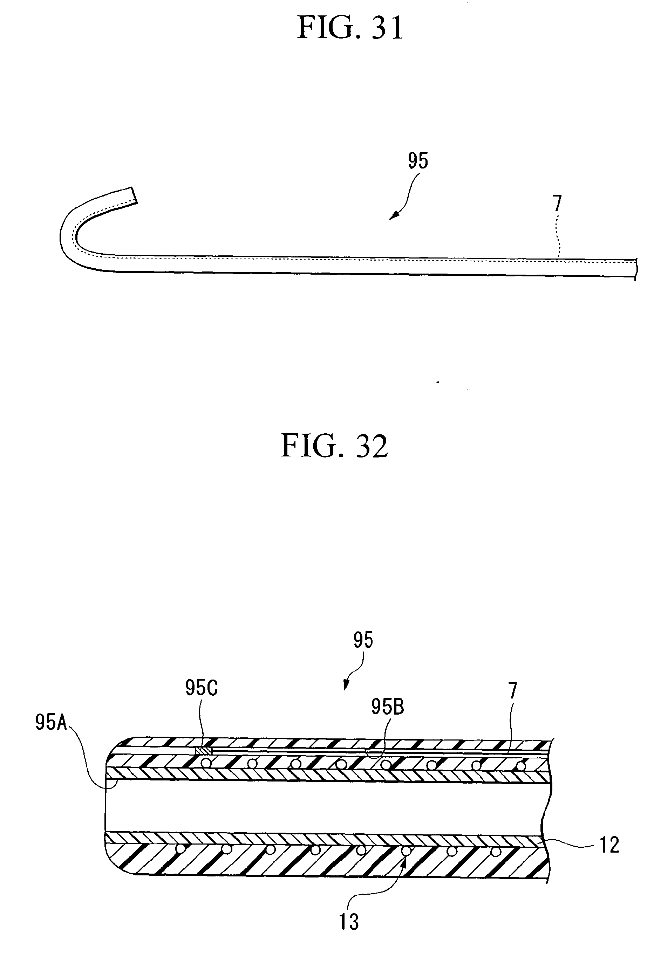

Moreover, as shown in FIG. 31 and FIG. 32, while the operation wire 7 without an exposed portion 7A is inserted into a through hole 95B for a wire which is provided in the curvable catheter 95 separately from the through hole 95A, the distal end thereof may be fixed to a fixing portion 95C provided in the vicinity of the distal end of the curvable catheter 95 (about 0.5mm to 5.0mm from the distal end). Here, the reason for being in the vicinity of the distal end is that the distal end of the curvable catheter is tapered. In this case, the curvable catheter 95 can also be curved.

## Claims

1. A treatment tool inserting/withdrawing auxiliary device comprising:
a catheter (6) through which a treatment tool (2) for an endoscope is to be inserted, and which is insertable into a channel (5) of a flexible endoscope (3);
an operation wire (7) which is inserted into the catheter (6), and is supported on a distal end or the vicinity of the catheter (6);
an operation portion (8) which moves the operation wire (7) back and forth with respect to the catheter (6); and
a curvable portion (16) which is provided on the catheter (6) and is curved by a back and forth movement of the operation wire (7), wherein
a coil layer (13A) is arranged at least on the curvable portion (16) of the catheter (6), and
the inner diameter of the catheter (6) is 1.5mm to 5.7mm,
**characterized in that**
the operation wire (7) is removable from the catheter (6) and **in that** one end of the operation wire (7) is connected to the operation portion (8), and the other end side is folded on the distal end of the catheter (6) and arranged toward the proximal side of the catheter (6).

2. The treatment tool inserting/withdrawing auxiliary device according to claim 1, wherein a pitch on the proximal side of the coil layer (13A) is greater than a pitch of the distal side thereof.

3. The treatment tool inserting/withdrawing auxiliary device according to claim 2, wherein the pitch on the proximal side of the coil layer (13A) is 0.5mm to 0.6mm.

4. The treatment tool inserting/withdrawing auxiliary device according to claim 1, wherein the distal end of the catheter (6) is provided with an index (62) at least a part of which is formed to be gradually wider toward the proximal side.

5. The treatment tool inserting/withdrawing auxiliary device according to claim 4, wherein the index (62) is formed in an approximate isosceles triangle.

6. The treatment tool inserting/withdrawing auxiliary device according to claim 1, wherein the curvable portion (16) is curvable within a range between 0 to 170 degree of a curved angle, assuming that the curved angle is an angle defined by axial centers of the proximal end and the distal end of the curvable portion (16), and the curved angle becomes 0 when the axial centers of the proximal end and the distal end are approximately on a same line having the curvable portion (16) therebetween.

7. The treatment tool inserting/withdrawing auxiliary device according to claim 1, wherein a surface of the proximal side of the catheter (6) is provided with convex portions or concavities.

8. The treatment tool inserting/withdrawing auxiliary device according to claim 7, wherein the catheter (6) has a hydrophilic lubricant surface.

9. The treatment tool inserting/withdrawing auxiliary device according to claim 1, wherein a mesh pipe layer (13B) is continually provided on the proximal end of the coil layer (13A), and arranged on the catheter (6).

## Patentansprüche

1. Hilfsvorrichtung zur Einführung/Entnahme eines Behandlungswerkzeugs, aufweisend:
einen Katheter (6), durch den ein Behandlungswerkzeug (2) für ein Endoskop einzuführen ist und das in einen Kanal (5) eines flexiblen Endoskops (3) eingeführt werden kann;
einen Betätigungsdraht (7), der in den Katheter (6) eingeführt wird und der am distalen Ende oder nahe des Katheters (6) befestigt ist;
einen Betätigungsabschnitt (8), der den Betätigungsdraht (7) relativ zum Katheter (6) vor und zurück bewegt; und
einen krümmbaren Abschnitt (16), der am Katheter (6) vorgesehen ist und durch die Vor- und Zurückbewegung des Betätigungsdrahtes (7) gekrümmt wird, wobei
eine Spulenlage (13A) zumindest auf dem krümmbaren Abschnitt (16) des Katheters (6) angeordnet ist, und
der Innendurchmesser des Katheters (6) 1,5 mm bis 5,7 mm beträgt,
**dadurch gekennzeichnet, dass**
der Betätigungsdraht (7) aus dem Katheter (6) entfernbar ist und dass ein Ende des Betätigungsdrahtes (7) mit dem Betätigungsabschnitt (8) verbunden ist und die andere Endseite am distalen Ende des Katheters (6) gefaltet und zur proximalen Seite des Katheters (6) angeordnet wird.

2. Hilfsvorrichtung zur Einführung/Entnahme eines Behandlungswerkzeugs nach Anspruch 1, wobei die Steigung an der proximalen Seite der Spulenlage (13A) größer ist als die Steigung an ihrer distalen Seite.

3. Hilfsvorrichtung zur Einführung/Entnahme eines Behandlungswerkzeugs nach Anspruch 2, wobei die Steigung an der proximalen Seite der Spulenlage (13A) 0,5 mm bis 0,6 mm beträgt.

4. Hilfsvorrichtung zur Einführung/Entnahme eines Behandlungswerkzeugs nach Anspruch 1, wobei das distale Ende des Katheters (6) mit einer Markierung (62) versehen ist, von der zumindest ein Teil zur proximalen Seite allmählich breiter wird.

5. Hilfsvorrichtung zur Einführung/Entnahme eines Behandlungswerkzeugs nach Anspruch 4, wobei die Markierung (62) etwa die Form eines gleichschenkligen Dreiecks hat.

6. Hilfsvorrichtung zur Einführung/Entnahme eines Behandlungswerkzeugs nach Anspruch 1, wobei der krümmbare Abschnitt (16) innerhalb eines Bereichs zwischen 0 und 170° eines Krümmungswinkels gekrümmt werden kann, wobei angenommen wird, dass der Krümmungswinkel definiert wird durch die axialen Mittelpunkte des proximalen Endes und des distalen Endes des krümmbaren Abschnitts (16) und der Krümmungswinkel 0 wird, wenn die axialen Mittelpunkte des proximalen Endes und des distalen Endes etwa auf derselben Linie mit dem krümmbaren Abschnitt (16) dazwischen liegen, dass das Führungselement in die Fixierposition verfahren ist.

7. Hilfsvorrichtung zur Einführung/Entnahme eines Behandlungswerkzeugs nach Anspruch 1, wobei die Oberfläche der proximalen Seite des Katheters (6) mit konvexen Abschnitten oder Vertiefungen versehen ist.

8. Hilfsvorrichtung zur Einführung/Entnahme eines Behandlungswerkzeugs nach Anspruch 7, wobei der Katheter (6) eine hydrophile Schmiermitteloberfläche hat.

9. Hilfsvorrichtung zur Einführung/Entnahme eines Behandlungswerkzeugs nach Anspruch 1, wobei eine kontinuierliche Siebrohrlage (13B) am proximalen Ende der Spulenlage (13A) und auf dem Katheter (6) angeordnet ist.

## Revendications

1. Dispositif auxiliaire d'insertion/retrait d'outil de traitement comprenant :
un cathéter (6) à travers lequel un outil de traitement (2) pour un endoscope doit être inséré, et qui est insérable à l'intérieur d'un canal (5) d'un endoscope flexible (3) ;
un fil d'actionnement (7) qui est inséré à l'intérieur du cathéter (6), et est supporté sur une extrémité distale ou à proximité du cathéter (6) ;
une partie d'actionnement (8) qui déplace le fil d'actionnement (7) vers l'arrière et vers l'avant par rapport au cathéter (6) ; et
une partie incurvable (16) qui est prévue sur le cathéter (6) et est incurvée par un mouvement vers l'arrière et vers l'avant du fil d'actionnement (7), dans lequel
une couche de bobinage (13A) est agencée au moins sur la partie incurvable (16) du cathéter (6), et
le diamètre intérieur du cathéter (6) est 1,5 mm à 5,7 mm,
**caractérisé en ce que**
le fil d'actionnement (7) est amovible du cathéter (6) et **en ce qu'**une extrémité du fil d'actionnement (7) est connectée à la partie d'actionnement (8), et l'autre côté d'extrémité est replié sur l'extrémité distale du cathéter (6) et agencé vers l'extrémité proximale du cathéter (6).

2. Dispositif auxiliaire d'insertion/retrait d'outil de traitement selon la revendication 1, dans lequel un pas sur le côté proximal de la couche de bobinage (13A) est supérieur à un pas du côté distal de celle-ci.

3. Dispositif auxiliaire d'insertion/retrait d'outil de traitement selon la revendication 2, dans lequel le pas sur le côté proximal de la couche de bobinage (13A) est 0,5 mm à 0,6 mm.

4. Dispositif auxiliaire d'insertion/retrait d'outil de traitement selon la revendication 1, dans lequel l'extrémité distale du cathéter (6) est prévue avec un index (62) dont au moins une partie est formée de façon à être graduellement plus large vers le côté proximal.

5. Dispositif auxiliaire d'insertion/retrait d'outil de traitement selon la revendication 4, dans lequel l'index (62) est formé comme un triangle approximativement isocèle.

6. Dispositif auxiliaire d'insertion/retrait d'outil de traitement selon la revendication 1, dans lequel la partie incurvable (16) est incurvable dans une plage entre 0 à 170 degré(s) d'un angle courbe, en supposant que l'angle courbe est un angle défini par des centres axiaux de l'extrémité proximale et de l'extrémité distale de la partie incurvable (16), et l'angle courbe devient zéro lorsque les centres axiaux de l'extrémité proximale et de l'extrémité distale sont approximativement sur une même ligne ayant la partie incurvable (16) entre ceux-ci.

7. Dispositif auxiliaire d'insertion/retrait d'outil de traitement selon la revendication 1, dans lequel une surface de l'extrémité proximale du cathéter (6) est prévue avec des parties convexes ou des concavités.

8. Dispositif auxiliaire d'insertion/retrait d'outil de traitement selon la revendication 7, dans lequel le cathéter (6) a une surface à lubrifiant hydrophile.

9. Dispositif auxiliaire d'insertion/retrait d'outil de traitement selon la revendication 1, dans lequel une couche de tuyau à maille (13B) est prévue de façon continue sur l'extrémité proximale de la couche de bobinage (13A), et agencée sur le cathéter (6).
